(19) Europäisches Patentamt
European Patent Office
Office européen des brevets

(11) Veröffentlichungsnummer: **0 390 021 A2**

# (12) EUROPÄISCHE PATENTANMELDUNG

(21) Anmeldenummer: **90105687.9**

(22) Anmeldetag: **26.03.90**

(51) Int. Cl.5: **C07C 45/51, C07C 49/213, C07D 317/54**

(30) Priorität: **30.03.89 DE 3910220**

(43) Veröffentlichungstag der Anmeldung: **03.10.90 Patentblatt 90/40**

(84) Benannte Vertragsstaaten: **BE CH DE FR GB LI NL**

(71) Anmelder: **BASF Aktiengesellschaft**
**Carl-Bosch-Strasse 38**
**D-6700 Ludwigshafen(DE)**

(72) Erfinder: **Naeumann, Fritz, Dr.**
**Rathenaustrasse 3a**
**D-6800 Mannheim 1(DE)**
Erfinder: **Hickmann, Eckhard, Dr.**
**Kantstrasse 23**
**D-6701 Dannstadt-Schauernheim(DE)**

(54) **Verfahren zur Herstellung von Benzylketonen.**

(57) Verfahren zur Herstellung von Benzylketonen der allgemeinen Formel I

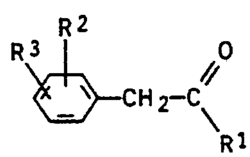

(I),

in der $R^1$ gegebenenfalls mit Cycloalkyl oder Aryl substituiertes $C_1$-$C_8$-Alkyl und $R^2$ und $R^3$ Halogen, $C_1$-$C_4$-Alkyl, Hydroxi, $C_1$-$C_4$-Alkoxi oder $C_2$-$C_4$-Methylendioxi bedeutet, indem man Glykolmonoether der allgemeinen Formel II

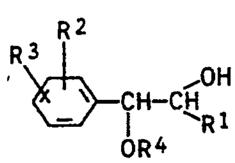

(II),

in der $R^1$, $R^2$ und $R^3$ die oben angegebenen Bedeutungen haben und in der $R^4$ für $C_1$-$C_8$-Alkyl steht, in Gegenwart von Siliciumdioxid, Phosphaten oder Zeolithen bei Temperaturen von 50 bis 500°C und Drücken von 0,01 bis 50 bar umsetzt.

EP 0 390 021 A2

## Verfahren zur Herstellung von Benzylketonen

Die Erfindung betrifft ein neues und verbessertes Verfahren zur Herstellung von Benzylketonen durch Kontakt von Glykolmonoethern mit Phosphaten, Siliciumdioxid oder Zeolithen.

Aus Houben-Weyl, Methoden der Organischen Chemie, Band VII/2a, Seite 931, 932, 968 und 981 ist die Herstellung von Benzylketonen durch Umlagerung von arylsubstituierten Oxiranen oder Glykolen an Homogenkatalysatoren wie kalter konzentrierter, heißer verdünnter Schwefelsäure, Eisessig, Phosphorsäure, Salzsäure und Acetylchlorid oder Heterogenkatalysatoren wie $SiO_2$, Aluminiumsilikate, zeolithe bekannt. Die Dehydratisierung von phenylierten Ethylenglykolen ist z.B. an mit $Fe_2O_3$, CaO und MgO dotierten amorphen Aluminiumsilikaten (Chem. Abstr. Vol. 82, 170 375) bzw. Ton (Chem. Abstr. Vol. 81, 120 220) in Suspension unter Druck in Ausbeuten von 50 bis 86 % zu Phenylacetaldehyden bekannt. Aus der US-A-2 444 400 ist die Dehydratisierung verschiedener Phenylethylenglykole durch saure Katalyse (z.B. Kieselgur, "silica", Phosphorsäure/Träger) bekannt. Als Einsatzprodukte werden auch Verbindungen mit Halogen, Alkoxy oder Alkyl substituiertem Phenylkern beschrieben. Chem. Abstr. Vol. 105, 190 662 enthält einen Hinweis auf die Verwendung von Aluminosilikatzeolithen vom Pentasiltyp bzw. Mordenit. Ferner ist aus US-A-2 628 255 bekannt, Styroloxide in der Gasphase zu Phenylacetaldehyd u.a. an Mg-Silikat umzulagern. Aus Chem. Abstr. Vol. 105, 190 662 ist die Umlagerung von Styroloxid an Zeolithen, auch des Pentasiltyps und aus Chem. Abstr. Vol. 81, 120 220 ist die Umlagerung von Styroloxide und Styrolglykole mit saurer Katalyse zu Aldehyden u.a. mit aktivierten Clays bekannt. Nachteilig an diesen Verfahren ist jedoch, daß man entweder auf die Oxirane, die wegen ihrer cyto- und/oder genotoxischen Eigenschaften besondere Sicherheitsmaßnahmen erforderlich machen, oder auf die Glykole, die aus den entsprechenden Oxiranen durch Hydrolyse hergestellt werden und die wegen ihrer guten Wasserlöslichkeit nur umständlich zu isolieren sind sowie wegen ihrer hohen Siedepunkte nur schwer verdampfbar sind, angewiesen ist.

Der vorliegenden Erfindung lag daher die Aufgabe zugrunde, ein neues und verbessertes Verfahren zur Herstellung von Benzylketonen zu finden und den Nachteilen der bekannten Verfahren abzuhelfen.

Demgemäß wurde ein neues und verbessertes Verfahren zur Herstellung von Benzylketonen der allgemeinen Formel I

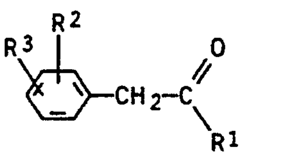 (I),

in der $R^1$ gegebenenfalls mit Cycloalkyl oder Aryl substituiertes $C_1$-$C_8$-Alkyl und $R^2$ und $R^3$ Halogen, $C_1$-$C_4$-Alkyl, Hydroxi, $C_1$-$C_4$-Alkoxi oder $C_2$-$C_4$-Methylendioxi bedeutet, gefunden, welches dadurch gekennzeichnet ist, daß man Glykolmonoether der allgemeinen Formel II

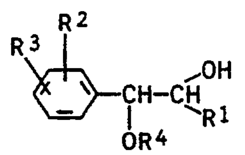 (II),

in der $R^1$, $R^2$ und $R^3$ die oben angegebenen Bedeutungen haben und in der $R^4$ für $C_1$-$C_8$-Alkyl steht, in Gegenwart von Siliciumdioxid, Phosphaten oder Zeolithen bei Temperaturen von 50 bis 500 °C und Drücken von 0,01 bis 50 bar umsetzt.

Die Benzylketone 1 sind nach folgender Methode erhältlich:

Die Umsetzung erfolgt durch thermische Behandlung von Glykolmonoethern II an Siliciumdioxid, Phosphaten oder Zeolithen unter Abspaltung von $R^4$-OH.

Die Reaktion kann sowohl in der Flüssigphase als auch diskontinuierlich oder vorzugsweise kontinuierlich in der Gasphase bei 50 bis 500 °C und 0,01 bis 50 bar durchgeführt werden.

Die Flüssigphasenreaktion kann beispielsweise als Suspensions-, Riesel-oder Sumpfreaktion bei Temperaturen von 50 bis 200 °C und Drücken von 0,5 bis 20 bar, vorzugsweise bei Temperaturen von 70 bis

170° C und Drücken von 1 bis 5 bar durchgeführt werden.

Die bevorzugte Gasphasenreaktion kann beispielsweise bei Temperaturen von 100 bis 500° C, vorzugsweise bei 150 bis 450° C und Drücken von 0,1 bis 50 bar, besonders bevorzugt bei 200 bis 400° C und Drücken von 0,5 bis 5 bar durchgeführt werden. Bei der Umsetzung in der Gasphase hält man vorteilhaft eine Katalysatorbelastung von 0,1 bis 20, insbesondere von 0,6 bis 5 g Ausgangsstoff der Formel II je g Katalysator und Stunde ein (WHSV = g Einsatzgemisch/g Katalysator und Stunde). Die Gasphasenreaktion kann in einem Festbett oder in einem Wirbelbett ausgeführt werden.

Das Verfahren wird im allgemeinen bei Normaldruck oder je nach Flüchtigkeit der Ausgangsverbindung bei vermindertem oder erhöhtem Druck vorzugsweise kontinuierlich durchgeführt.

Schwerflüchtige oder feste Ausgangsstoffe werden in gelöster Form z.B. in THF-, Toluol- oder Petrolether-Lösung eingesetzt. Allgemein ist auch eine Verdünnung mit diesen Lösungsmitteln oder Inertgasen wie $N_2$, Ar, $H_2O$-Dampf möglich.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt. Auch eine direkte Weiterverarbeitung der Reaktionsprodukte ist aufgrund der hohen Ausbeuten möglich.

Bevorzugt werden gasförmigen Reaktionsprodukte sofort in eine Trennung eingebracht und z.B. in einer Fraktionierkolonne in ihre Einzelkomponenten zerlegt.

Die Glykolmonoether II erhält man in technisch einfacher Weise durch Epoxidation der entsprechenden Styrole mit $H_2O_2$ unter "Payne-Bedingungen" (Organic Synthesis, 19.., 60, S. 63), z.B.

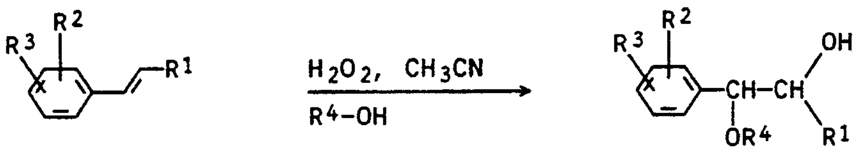


Die Substituenten in den Verbindungen der Formeln I und II haben insbesondere folgende Bedeutungen:

$R^1$ - unverzweigtes oder verzweigtes $C_1$- bis $C_8$-Alkyl, bevorzugt unverzweigtes oder verzweigtes $C_1$- bis $C_4$-Alkyl wie Methyl, Ethyl, n-Propyl, iso-Propyl, n-Butyl, iso-Butyl, sec.-Butyl und tert.-Butyl, bevorzugt Methyl und Ethyl,

$R^2$, $R^3$ unabhängig voneinander in ortho-, meta- und/oder para-Stellung stehende Substituenten

- Halogen wie Fluor, Chlor, Brom und Iod,
- unverzweigtes oder verzweigtes $C_1$- bis $C_4$-Alkyl wie Methyl, Ethyl, n-Propyl, iso-Propyl, n-Butyl, iso-Butyl, sec.-Butyl und tert.-Butyl, bevorzugt Methyl und Ethyl,
- Hydroxi,
- unverzweigtes oder verzweigtes $C_1$- bis $C_4$-Alkoxi wie Methoxi, Ethoxi, n-Propoxi, iso-Propoxi, n-Butoxi, iso-Butoxi, sec.-Butoxi und tert.-Butoxi, bevorzugt Methoxi und Ethoxi, sowie 3-Methoxi-4-hydroxi,
- Methylendioxi, bevorzugt Methylendioxigruppe, in 3,4-Position am Phenylring,

$R^4$ - unverzweigtes oder verzweigtes $C_1$- bis $C_8$-Alkyl, bevorzugt unverzweigtes oder verzweigtes $C_1$- bis $C_4$-Alkyl wie Methyl, Ethyl, n-Propyl, iso-Propyl, n-Butyl, iso-Butyl, sec.-Butyl und tert.-Butyl, bevorzugt Methyl und Ethyl.

Als Glykolmonoether II für das erfindungsgemäße Verfahren sind folgende bevorzugt geeignet:

1-Methoxi-1-phenyl-propan-2-ol,
1-Methoxi-1-(4-fluorphenyl)-propan-2-ol,
1-Methoxi-1-(2-chlorphenyl)-propan-2-ol,
1-Methoxi-1-(4-methylphenyl)-propan-2-ol,
1-Methoxi-1-(4-methoxiphenyl)-propan-2-ol,
1-Methoxi-(3,4-methylendioxiphenyl)-propan-2-ol,
1-Methoxi-(3-methoxi-4-hydroxi)-propan-2-ol.

Als bevorzugte Produkte I des erfindungsgemäßen Verfahrens erhält man dementsprechend:

1-Phenylpropan-2-on,
1-(4-Fluorphenyl)-propan-2-on,
1-(2-Chlorphenyl)-propan-2-on,
1-(4-Methylphenyl)-propan-2-on,
1-(4-Methoxiphenyl)-propan-2-on,

1-(3,4-Methylendioxiphenyl)-propan-2-on,
1-(3-Methoxi-4-hydroxi)-propan-2-on.

Als Katalysatoren für die erfindungsgemäße Verfahren zur Herstellung von Benzylketonen eignen sich Zeolithe, besonders acide Zeolithe. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1 : 2. Die Elektrovalenz der Aluminium enthaltenden Tetraeder ist durch Einschluß von Kationen in den Kristall, z.B. eines Alkali- oder Wasserstoffions ausgeglichen. Ein Kationaustausch ist möglich. Die Räume zwischen den Tetraedern sind vor der Dehydration durch Trocknen bzw. Calcinieren von Wassermolekeln besetzt.

In den Zeolithen können anstelle von Aluminium auch andere drei- und zweiwertige Elemente wie B, Ga, Fe, Cr, Be, As, Sb in das Gitter eingebaut werden oder das Silicium kann durch ein vierwertiges Element wie Ge, Ti, Zr, Hf, ersetzt werden.

Als Katalysatoren kommen Zeolithe aus der Mordenit-Gruppe oder Faujasit-Gruppe wie L-Zeolith oder engporige Zeolithe vom Erionit- bzw. Chabasit-Typ in Betracht. Besonders vorteilhaft für das erfindungsgemäße Verfahren sind Zeolithe vom Pentasil-Typ. Diese Zeolithe können unterschiedliche chemische Zusammensetzung aufweisen. Es handelt sich hierbei um Alumino-, Boro-, Eisen-, Gallium-, Chrom-, Beryllium-, Arsen-, Antimon- und Wismutsilikatzeolithe oder deren Gemische sowie um Alumino-, Boro-, Gallium- und Eisengermanatzeolithe oder deren Gemische.

Insbesondere eignen sich die Alumino-, Boro-, Gallium- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck hergestellt. Geeignet sind auch die isotaktischen Zeolithe nach DE-A-3 006 471. Die erhaltenen Aluminosilikatzeolithe weisen je nach Wahl der Einsatzstoffmenge ein $SiO_2/Al_2O_3$-Verhältnis von 10 bis 40.000 auf und können auch in etherischem Medium z.B. im Diethylenglykoldimethylether, in alkoholischem Medium wie Methanol bzw. 1,4-Butandiol oder in Wasser synthetisiert werden.

Die Borosilikatzeolithe unter Einschluß der isotaktischen Borosilikatzeolithe können z.B. bei 90 bis 200°C unter autogenem Druck synthetisiert werden, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit oder insbesondere ohne Alkali- oder Erdalkalizusatz zur Reaktion bringt. Die Borosilikatzeolithe können auch statt in wäßriger Aminlösung in etherischer Lösung, z.B. die Ehylenglykoldimethylether oder in alkoholischer Lösung, z.B. 1,6-Hexandiol hergestellt werden.

Eisensilikatzeolithe erhält man z. B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220°C unter autogenem Druck.

Die so hergestellten Alumino-, Boro- und Eisensilikatzeolithe können nach ihrer Isolierung, Trocknung bei 100 bis 160° C, vorzugsweise 110°C und Calcinierung bei 450 bis 550°C, vorzugsweise 500 bis 540°C, mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 95 : 5, bevorzugt 75 : 25, Siliciumdioxid, bevorzugt hochdisperses $SiO_2$, Gemische aus hochdispersem $SiO_2$ und hochdispersem $Al_2O_3$ sowie Ton. Nach der Verformung werden die Extrudate oder Preßlinge bei 110°C/16 h getrocknet und bei 500°C/16 h calciniert.

Man kann auch die isolierten Alumino- bzw. Borosilikatzeolithe direkt nach der Trocknung verformen und erstmals nach der Verformung einer Calcinierung unterwerfen. Die Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Kieselsäureester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die zeolithe durch Abbrennen der Koksablagerung mit Luft oder mit einem Luft/$N_2$-Gemisch bei 400 bis 550°C, bevorzugt 500 bis 540°C, zu regenerieren. Die Zeolithe erhalten dadurch ihre Anfangsaktivität zurück. Durch partielle Verkokung (pre-coke) ist es möglich, die Aktivität des Katalysators für ein Selektivitätsoptimum des gewünschten Reaktionsproduktes einzustellen.

Um eine möglichst große Selektivität, hohen Umsatz sowie lange Standzeiten zu erreichen, ist es mitunter vorteilhaft, die Zeolithe zu modifizieren. Eine geeignete Modifizierung der Katalysatoren besteht z.B. darin, daß man die unverformten oder verformten Zeolithe mit Metallsalzen durch einen Ionenaustausch oder durch Imprägnierung dotiert.

Zweckmäßigerweise führt man die Dotierung so durch, daß man die verformten Zeolithe in einem Steigrohr vorlegt und bei 20 bis 100°C eine wäßrige oder ammoniakalische Lösung eines Halogenids oder eines Nitrats der Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf Zeolithe besteht darin, daß man das zeolithische Material z. B. mit einem Halogenid, einem Nitrat oder einem Oxid der Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft.

Zu den verwendbaren siliciumreichen Zeolithen ($SiO_2/Al_2O_3 \geqq 10$) gehören auch die bekannten ZSM-Typen sowie Ferrierit und Nu-1 sowie Silicate® ein Molekularsieb, ein sog. Silica Polymorph.

Weitere Katalysatoren für die Herstellung von Benzylketone 1 aus entsprechenden Glycolmonoethern II werden im folgenden beschrieben: Als Aluminiumphosphat-Katalysatoren werden für das erfindungsgemäße Verfahren insbesondere unter hydrothermalen Bedingungen synthetisierte Aluminiumphosphate eingesetzt. Die unter hydrothermalen Bedingungen hergestellten Aluminiumphosphate sind z.B. AlPO-5, AlPO-9, AlPO-11, AlPO-12, AlPO-14, AlPO-21, AlPO-25, AlPO-31 und AlPO-33. Synthesen dieser Verbindungen sind in EP-A-132 708, US-A 4 310 440 und US-A 4 473 663 beschrieben.

AlPO-5 kann man beispielsweise synthetisieren, indem man Orthophosphorsäure mit Pseudoboehmit (Catapal SB®) in Wasser homogen mischt, zu dieser Mischung Tetrapropylammoniumhydroxyd gibt und danach bei etwa 150°C 20 bis 60 h unter autogenem Druck in einem Autoklaven umsetzt. Das abfiltrierte AlPO-5 bei 100 bis 160°C trocknet und bei 450 bis 550°C calciniert. AlPO-9 kann aus Orthophosphorsäure und Pseudoboehmit in wäßriger DABCO-Lösung (1,4-Diazabicyclo-(2,2,2)-octan) bei etwa 200°C unter autogenem Druck während 200 bis 400°C synthetisiert werden. Die Synthese des AlPO-21 erfolgt aus Orthophosphorsäure und Pseudoboehmit in wäßriger Pyrrolidon-Lösung bei 150 bis 200°C unter autogenem Druck während 50 bis 200 h.

Die für das erfindungsgemäße Verfahren eingesetzten Siliciumaluminiumphosphate sind z.B. SAPO-5, SAPO-11, SAPO-31 und SAPO-34. Die Synthese dieser Verbindung ist in EP-PS 103 117 und US-A 4 440 871 beschrieben. Man erhält diese Stoffe durch Kristallisation aus wäßriger Mischung bei 100 bis 250°C unter autogenem Druck während 2 h bis 2 Wochen, wobei die Reaktionsmischung aus einer Silicium-, Aluminium- und Phosphorkomponente in wäßrigen aminoorganischen Lösungen umgesetzt wird. SAPO-5 beispielsweise wird durch Mischung von $SiO_2$, suspendiert in wäßriger Tetrapropylammoniumhydroxid-Lösung, mit einer wäßrigen Suspension aus Pseudoboehmit und Orthophosphorsäure und anschließende Umsetzung bei 150 bis 200°C während 20 bis 200 h unter autogenem Druck in einem Rührautoklaven erhalten. Die Trocknung des abfiltrierten Pulvers erfolgt bei 110 bis 160°C und die Calcination bei 450 bis 550°C. Als Siliciumaluminiumphosphate sind z.B. auch ZYT-5, ZYT-6, ZYT-7, ZYT-9, ZYT-11 und ZYT-12 geeignet (JP 59/217-619).

Borphosphate für das erfindungsgemäße Verfahren kann man durch Mischen und Kneten von konzentrierter Borsäure und Phosphorsäure und durch anschließende Trocknung und Calcination in Inertgas-, Luft- oder Dampfatmosphäre bei 250 bis 650°C, vorzugsweise 300 bis 500°C, herstellen. Geeignete Phosphate sind z.B. auch $CePO_4$, $FePO_4$ und $Zr_3(PO_4)_4$. Auch Siliciumdioxid oder Aluminiumoxid kann als Katalysator eingesetzt werden.

Die Katalysatoren können wahlweise als 2- bis 4-mm-Stränge oder als Tabletten mit 3 bis 5 mm Durchmesser oder als Pulver mit Teilchengrößen von 0,1 bis 0,5 mm oder als Wirbelkontakt eingesetzt werden.

Die nach dem erfindungsgemäßen Verfahren zugänglichen Benzylketone I sind wichtige zwischenprodukte zur Herstellung von Aminen oder Alkoholen, sowie zur Synthese von Wirkstoff- und Riechstoffzwischenprodukten.

Herstellung der Katalysatoren:

Katalysator A

Ein Aluminosilikatzeolith vom Pentasil-Typ wird unter hydrothermalen Bedingungen bei autogenem Druck und 150°C aus 650 g hochdispersem $SiO_2$, 203 g $Al_2(SO_4)_3 \cdot 18\ H_2O$ in 10 kg einer wäßrigen 1,6-Hexandiamin-Lösung (Mischung 50 : 50 Gew.-%) in einem Rührautoklaven hergestellt. Nach Abfiltrieren und Auswaschen wird das kristalline Reaktionsprodukt bei 110°C/24 h getrocknet und bei 500°C/24 h calciniert. Der Aluminosilikatzeolith enthält 92,8 Gew.-% $SiO_2$ und 4,2 Gew.% $Al_2O_3$.

Katalysator A erhält man, indem man den reinen Aluminosilikatzeolith mit einem Verformungshilfsmittel zu 2-mm-Strängen verformt, bei 110°C/16 h trocknet und bei 500°C/24 h calciniert.

Katalysator B

Ein Borosilikatzeolith des Pentasil-Typs wird in einer hydrothermalen Synthese aus 640 g hochdispersem $SiO_2$, 122 g $H_3BO_3$, 8 kg einer wäßrigen 1,6-Hexandiamin-Lösung (Mischung 50 : 50 Gew.-%) bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. Dieser Borosilikatzeolith setzt sich zusammen aus 94,2 Gew.-% $SiO_2$ und 2,3 Gew.-% $B_2O_3$.

Mit diesem Material werden durch Verformen mit einem Verformungshilfsmittel 2-mm-Stränge hergestellt, die bei 110°C/16 h trocknet und bei 500°C/24 h calciniert werden.

Beispiele 1 bis 4

Die Reaktionen werden unter isothermen Bedingungen in einem Rohrreaktor (0,6 cm, 50 cm Länge) in der Gasphase mindestens 6 Stunden lang durchgeführt. Die Trennung und Charakterisierung der Reaktionsprodukte erfolgt nach üblichen Methoden. Die quantitative Bestimmung der Reaktionsprodukte und der Ausgangsstoffe erfolgt gaschromatographisch.

Die Versuchsergebnisse sind in der nachfolgenden Tabelle zusammengestellt.

Tabelle

Einsatzstoff: 1-Methoxi-1-(3,4-methylendioxiphenyl)-propan-2-ol
Produkt: 1-Methoxi-1-(3,4-methylendioxiphenyl)-propan-2-on

| Beispiel | 1 | 2 | 3 | 4 |
|---|---|---|---|---|
| Katalysator | A | A | B | B |
| Temperatur [°C] | 250 | 300 | 250 | 300 |
| WHSV [$h^{-1}$] | 1.9 | 2.1 | 1.8 | 2.2 |
| Umsatz [%] | 99 | 99 | 99 | 99 |
| Selektivität Wertprodukt [%] | 95.2 | 93.5 | 90.3 | 89.2 |

## Ansprüche

1. Verfahren zur Herstellung von Benzylketonen der allgemeinen Formel I

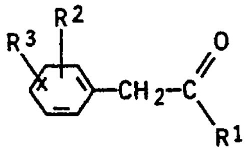

(I),

in der $R^1$ gegebenenfalls mit Cycloalkyl oder Aryl substituiertes $C_1$-$C_8$-Alkyl und $R^2$ und $R^3$ Halogen, $C_1$-$C_4$-Alkyl, Hydroxi, $C_1$-$C_4$-Alkoxi oder $C_2$-$C_4$-Methylendioxi bedeutet, dadurch gekennzeichnet, daß man Glykolmonoether der allgemeinen Formel II

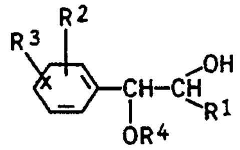

(II),

in der $R^1$, $R^2$ und $R^3$ die oben angegebenen Bedeutungen haben und in der $R^4$ für $C_1$-$C_8$-Alkyl steht, in Gegenwart von Siliciumdioxid, Phosphaten oder Zeolithen bei Temperaturen von 50 bis 500°C und Drücken von 0,01 bis 50 bar umsetzt.

2. Verfahren nach Anspruch 1, dadurch gekennzeichnet, daß man als Katalysatoren Aluminiumsilikat-, Borosilikat-, Galliumsilikat- oder Eisensilikatzeolithe des Pentasil-Typs verwendet.

3. Verfahren nach Anspruch 1, dadurch gekennzeichnet, daß man als Phosphate Aluminiumphosphat, Siliciumaluminiumphosphat, Eisenaluminiumphosphat, Eisensiliciumaluminiumphosphat oder Borphosphat einsetzt.

4. Verfahren nach Anspruch 1, dadurch gekennzeichnet, daß man als Katalysator Siliciumdioxid einsetzt.

5. Verfahren nach Anspruch 1, dadurch gekennzeichnet, daß man die Umsetzung in der Gasphase bei 100 bis 500°C und 0,01 bis 50 bar durchführt.